# EUROPEAN PATENT APPLICATION

(11) **EP 1 226 787 A2**
(43) Date of publication of application: **31.07.2002**
(21) Application number: 02001860.2
(22) Date of filing: 28.01.2002
(51) Int. Cl.: A61B 18/20

(54) **Device for cutaneous treatments using optical radiation**

(30) Priority: 29.01.2001 IT MO010008
(71) Applicant: Laserwave S.r.l., 41100 Modena (IT)
(72) Inventor: Meloni, Gian Paolo, 41100 Modena (IT)
(74) Representative: Guareschi, Antonella

(57) **Abstract**

The invention falls into the field of devices designed to enable cutaneous treatments involving optical radiation. An optical cooling unit (1) is composed of a container with two sides (3) and (4) opposite each other, parallel to each other and spaced out by a cavity (2). The said two sides are composed, for the main part of their surface, of two optically transparent elements (3a) and (4a). The cavity (2) is designed to enable the continual circulation of a cooling liquid. The optical cooling unit (1) is attached integrally to a device (7) and it is crossed, at a substantially orthogonal angle, by optical radiation (10). Additional optics (15), reciprocally interchangeable, may be inserted in a seating (14).

## Description

The invention relates to a device for cutaneous treatments.

More and more applications are being found for optical radiation, using for example, laser rays, for both operations aimed at eliminating or reducing cutaneous defects of a vascular nature and also those designed to eliminate the presence of hairs in particular areas of the skin.

In the case of the aforesaid defects of a vascular nature, these are usually small superficial lesions or reticules of the epidermal capillaries which are particularly visible and cutaneous marks due to angioma constituted of dilated, tortuous vessel elements which are generally wine red in colour and have an anomalous structure; the said cutaneous defects are mainly located in particularly visible areas of the anatomy, such as the face, neck and lower limbs.

Still with regards to the cutaneous defects, a further example could be the marks which appear with the progression of the ageing process.

Of the other types of cutaneous illness that are treatable with optical radiation, psoriasis (characterised by circumscribed patches with a red background covered with whitish scales) is worth a particular mention.

Also worth mentioning are tattoos, the elimination of which can be performed using cutaneous treatments involving optical radiation. In fact, optical radiation determines the carbonisation of the cells which have absorbed the colourings and the said cells, after their carbonisation, can be eliminated by the blood circulation.

Operations designed to eliminate or limit these defects are of a medical nature which are not disassociated, as explained earlier, from aspects of a cosmetic nature.

Operations designed to remove hair from a particular area of the body are pre-eminently of a cosmetic nature and to understand fully the importance of these treatments, one must simply consider the fact that the presence of hairs in particularly visible areas of the body can lead to serious psychological problems for the person concerned.

The use of optical radiation, in particular laser rays, has led to the realisation of devices capable of dealing successfully with the aforesaid cutaneous defects, both those linked to a defect of a vascular nature and those linked to an excess of hairs.

The commonly known devices all use, substantially, a group of common elements. In fact, there is always a radiating element, a conveyor for the radiation towards one or more condensation lenses of the said radiation and a terminal optical element or unit, commonly designed to be positioned in contact with the cutaneous surface to be treated.

One problem which is common to all the commonly known kinds of devices for cutaneous treatments which use optical radiation is constituted of the cooling of the cutaneous portion on which the treatment is to be carried out on.

This cooling is important both to produce a localised reduction in the cutaneous temperature and also to produce an always localised absorption of the increase in temperature due to the application of the optical radiation.

The cooling during the treatment has, substantially, both the aim of anaesthetising the part affected by the application of optical radiation and also that of preventing burns and/or denaturing of the skin.

The cooling after the treatment also has a dual purpose: to absorb the accumulation of the heat caused by the application of the optical radiation and to limit the reddening of the skin.

In order to obtain the aforesaid cooling, the commonly known technique offers various solutions among which the first and most simple is the application of cryogenic products in the form of a spray or ice applied directly to the epidermis before the application of the optical radiation.

There are numerous drawbacks to the application of these skin-cooling products: first of all, the application of these products does not coincide with the application of the optical radiation; furthermore, where the cryogenic products in particular are concerned, their extreme volatility must be considered as it causes the cooled part to remain cool for an extremely short space of time only with the consequent cooling effect greatly reduced at the moment of the treatment; furthermore, one should also consider the limits of the depth reached by the cooling applied in this way and also the lack of constancy of the cooling times and, in any case, the reduced ease of use.

The application of the aforesaid cooling products may be performed not only directly onto the portion of the epidermis to undergo the treatment but also on the optical element that will come into contact with the said portion of the epidermis and through which the optical radiation will cross: an example of this can be found in the document US-6,015,404.

A further known solution is constituted of the direct cooling of the said optical element located in direct contact with the epidermis obtained via the conduction principle: in this solution, the said optical element is brought into contact with a metal element kept at a low temperature.

The major drawback of this means of bringing about the cutaneous cooling process is constituted of the mediocre results achieved.

An optical element has been proposed which is cooled by a cooling fluid circulating inside a containing frame of the said optical element. This solution has the drawback that the cooling of the optical element is not a uniform process as the central part of the said element, being further from the said frame, is always at a higher temperature than the peripheral areas nearer the said frame.

The cooling fluid can be water, water mixed with liquid coolants or even gases such as carbon dioxide, freon and suchlike.

An example of the cooling, with gas, of the optical unit designed to be placed on the epidermis to be treated can be found in the document US-5,344,418.

Lastly, a further proposal for a device equipped with a scanner capable of moving the optical radiation over a larger cutaneous surface than the surface affected by the fixed optic with which the majority of the commonly known devices are equipped; in this way every time the device is placed on the epidermis, the treatment performed covers a larger surface area.

The most advantageous, and therefore the most desirable, characteristic of a device for superficial cutaneous treatments using optical radiation can be resumed as follows: uniformity and certainty of the cooling of both the optical element in contact with the epidermis and the epidermis itself, the amplitude of the surface affected by the treatment, the irrelevance of the shape of the epidemic surface to be treated and the irrelevance of the operator's manual expertise.

Every single one of the commonly known devices has only a few of the most advantageous characteristics mentioned above. None of them seems to have them all simultaneously.

A first aim of this invention is to realise an optical cooling unit which is particularly efficient in the cooling action it performs on the skin and is equipped with an operating surface (through which the optical radiation can pass) capable of guaranteeing an extremely large treatment zone.

A further aim of this invention is to realise a device for superficial cutaneous treatments by means of the use of optical radiation, in particular, laser rays, which has all the most advantageous characteristics listed above at the same time.

A still further aim of this invention is to permit the immediate use of the said device in the performing of cutaneous treatments of a variety of kinds in which a different imprinting of the optical radiation is required on the epidermis, said imprinting being more or less ample depending on the case.

In particular, the device for cutaneous treatments using optical radiation, in particular laser rays, which can be held by hand and comprising a conductor for the aforesaid optical radiation, an optic designed to condense the said radiation, at least one scanner designed to deflect optical radiation, at least one reflective mirror and an optical cooling unit attached integrally to the device, is characterised by the fact that the optical cooling unit is constituted of a container which is substantially parallelepiped in form and is fitted with two openings, identical in size and shape, created on both sides of the said container, the two said sides being opposite each other, parallel to each other and spaced out by a cavity; inside each of the said openings is an optically transparent element whose surfaces and dimensions are identical to that of the respective opening; the optical cooling unit being attached integrally to the device in such a way as to ensure both optically transparent elements are substantially orthogonal to the direction of the optical radiation; the cavity being designed to enable the circulation of a cooling fluid; and which is equipped with a seating designed to enable the insertion of one of a plurality of additional optics at a time, said additional optics being interchangeable and designed to be positioned inside the device in such a way that they are crossed by optical radiation; the additional optics being designed to vary the focalisation of the said optical radiation on the epidermis.

This and other characteristics will better emerge from the detailed description that follows of a preferred embodiment, provided in the form of a non-limiting example, with reference to the accompanying drawings, in which:

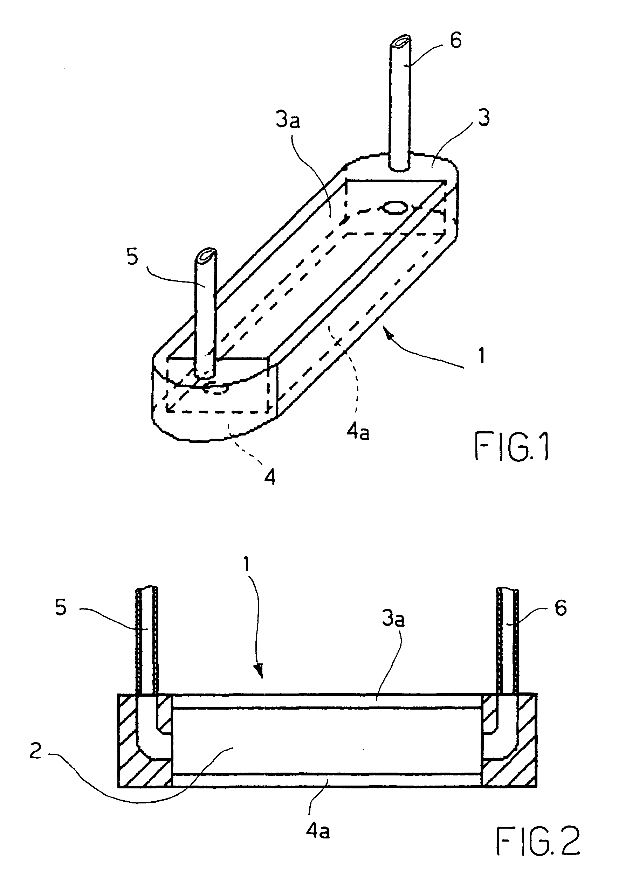

- Figure 1 shows a perspective view of the optical cooling unit;
- Figure 2 shows a view of a lateral longitudinal section of the optical cooling unit;

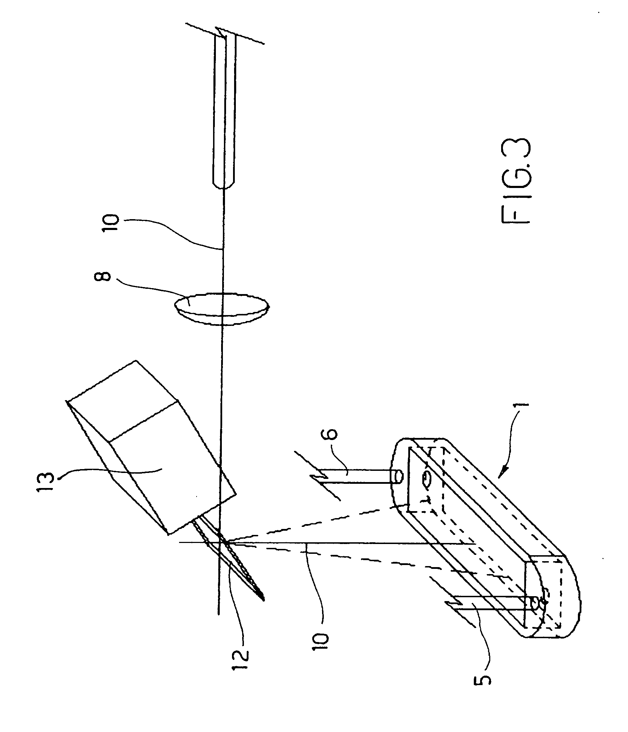

- Figure 3 shows a perspective view of the unit composed of all the main elements constituting the device;

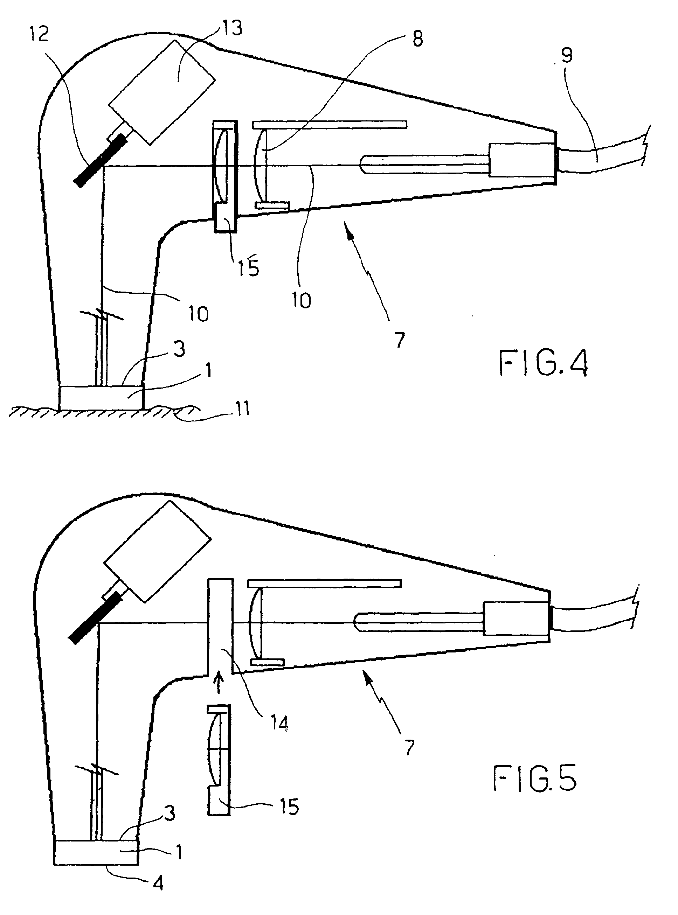

- Figure 4 shows a lateral transparent view of a possible embodiment of the device;
- Figure 5 shows the same elements as figure 4 with the additional optic seating highlighted;

With reference to the figures, 1 indicates an optical cooling unit with a substantially parallelepiped form, constituting a container.

The said container has two sides 3 and 4 opposite and parallel to each other and spaced out by a cavity 2; each of the said sides has an opening in which a transparent optical element is positioned, respectively 3a and 4a. The surface of each of the said openings occupies almost the totality of the surface of the respective side in which it is located.

The form of the aforesaid opening and consequently of the respective transparent optical elements 3a and 4a is long and their surface is no less than 250mm².

Two orifices 5 and 6 are positioned on the side 3 in position with the portion of the said side which is not occupied by the transparent optical element 3a.

The optical cooling element 1 is attached integrally to a device 7 containing an optic 8 connected to a conductor 9, in the example illustrated constituted of a fibre optic cable, connected to an external optical radiation 10 generator (not illustrated), generating, in particular, a laser ray.

The two transparent optical elements 3a and 4a can be made of either the same material or a different material: in the latter case, for example, the element 3a, positioned facing the interior of the device 7 can be made of glass while the element 4a, positioned facing the epidermis 11 to be treated can be made of sapphire, quartz, industrial diamond or suchlike.

In front of the optic's 8 radiation 10 output is a mirror 12 inclined in relation to the said radiation. The said mirror is connected to a scanner 13.

On the external body of the device 7 is a seating 14 designed to enable the insertion of an additional optic 15 which is part of a plurality; all the additional optics making up the said plurality are reciprocally interchangeable.

The additional optic 15 is positioned, inside the device 7, in position with the optic's 8 radiation 10 output, between this latter and the mirror 12.

Each of the aforesaid optics 15 has a different focalising power.

The functioning modes of the present invention will now be described with reference to the indications in the figures.

The device 7 is held by the operator and placed on the patient's epidermis 11 in position with the cutaneous zone to be treated. The contact between the said device and the said epidermis is performed by means of the transparent optical element 4a, preferably made of a material such as sapphire or industrial diamond or a similar material with a greater level of thermal conductivity than common glass.

The cooling fluid which flows continuously within the cavity 2, entering the said cavity through the orifice 5 and leaving it via the orifice 6, keeps both the transparent optical elements 3a and 4a, and consequently also the portion of epidermis 11 in contact with the element 4a, at a low temperature.

The said cooling action is exerted before, during and after the cutaneous treatment by means of the optical radiation 10, in particular a laser ray. The said treatment, thanks to the ample surface of the transparent optical elements 3a and 4a, allows for the use of optical radiation 10, which, thanks to the scanner 13, is deflected along the aforesaid elements.

The pressure applied by the operator onto the device 7 is transmitted onto the skin in position with the contact between the said skin and the transparent optical element 4a; said pressure leading to the dilation of the pores and increasing tautness of the skin, facilitates the transmission of the cooling effect to the said skin with the consequent increased depth of the penetration of the said cooling effect through the thickness of the skin.

The aforesaid pressure exerted by the transparent optical element 4a on the skin also leads to a reduction in the distance between the optical cooling unit 1 and the capillaries or piliferous bulbs or cells to be treated.

Thanks to the uniform cooling of the element 4a and thanks also to the long form of both the transparent optical elements 3a and 4a, the application of the cutaneous treatment is also possible in anatomical zones that are neither flat nor regular in shape and, so, cannot be reached by the commonly known types of device.

Depending on the dimensions of the imprinting of the radiation 10 on the epidermis 11, the said dimensions being a variable which depends on the cutaneous operation required, one inserts the additional optic 15 with the focalisation power required into the device 7, making use of the relevant seating 14.

The said additional optic is part of a plurality and all the optics belong to the said plurality, while having different focalisation powers, are reciprocally interchangeable.

It should also be noted that the presence of the scanner 13, deflecting the optical radiation 10, allows the operator to treat a larger zone of skin with a constant energy distribution, without moving the device 7. In this way localised accumulations of energy are prevented and consequently so too is cutaneous damage.

A first advantage offered by the device in question in this invention lies in the fact that the cooling system of the optical cooling unit 1 provides certainty and uniformity in the cooling action exerted on the cutaneous zone to be treated.

A further advantage lies in the fact that, thanks to the form and the dimensions of the transparent optical elements 3a and 4a, the device 7 can be used to treat any cutaneous zone, regardless of the shape of the said zone and regardless of the operator's manual ability.

A still further advantage offered by this invention lies in the size of the cutaneous surface that can be treated, the said surface being 250mm².

A still further advantage is that all the advantageous characteristics just mentioned are incorporated in a single device.

A further advantage lies in the possibility of obtaining, by means of the said device, different focalisations of the optical radiation on the epidermis by inserting additional optics into the said device, said optics being reciprocally interchangeable, thus obtaining greater flexibility of use with regards to the device in question in this invention.

In the description, specific reference has been made to the existence, inside the device in question in this invention, of a single scanner 13 and a single mirror 12, but it is clear that the number of these components could also be greater without reducing the said device's functioning efficiency.

## Claims

1. A device (7) for cutaneous treatments using optical radiation (10), in particular a laser ray, of a handheld kind and comprising, at least, one conductor (9) for the aforesaid optical radiation, at least one scanner (13) designed to deflect the optical radiation (10), at least one mirror (12) to reflect the said radiation, and an optical cooling unit (1) attached integrally to the device (7), **characterised by** the fact that the optical cooling unit (1) is constituted of a container which is substantially parallelepiped in form and has two openings, both identical in terms of dimensions and form, located on the two sides (3) and (4) of the said container, the said two sides being opposite each other and parallel and spaced out by a cavity (2); in each part of the said openings is placed a transparent optical element, respectively (3a) and (4a), with dimensions which are substantially identical to those of the respective opening; the optical cooling unit (1) being attached to the device (7) in such a way that both transparent optical elements (3a) and (4a) are crossed by the optical radiation (10) reflected by at least one mirror (12); the cavity (2) being designed to enable the circulation of a cooling fluid; and which is fitted with a seating (14) designed for the insertion of a single additional optic (15) at a time, said additional optic being part of a plurality of optics which are reciprocally interchangeable and are designed to be positioned inside the device (7) in such a way that the said optics are crossed by the optical radiation (10); the additional optics (15) being designed to vary the focalisation of the said optical radiation on the epidermis (11).

2. A device according to claim 1, **characterised by** the fact that the attachment between the optical cooling unit (1) and the device (7) is such that the transparent optical element (3a) is positioned facing the interior of the said device and the transparent optical element (4a) is positioned facing the exterior of the said device; the external surface of the element (4a) being designed to be placed on the portion of epidermis (11) to be treated.

3. A device according to claims 1 and 2, **characterised by** the fact that the transparent optical elements (3a) and (4a) both have a surface of at least 250mm² which occupies almost the totality of the surface of the respective side (3) and (4); the said dimensions being designed to permit, contemporaneously, both the deflection of the optical radiation (10) and the contact with the external surface of the transparent optical element (4a) even on portions of epidermis which are not flat and/or are irregular.

4. A device according to claims 1, 2 and 3, **characterised by** the fact that the two transparent optical elements (3a) and (4a) can be made of either the same material or different materials: in the case of the different materials, for example, the element (3a), positioned facing the interior of the device (7) can be made of glass, while the element (4a) positioned facing the epidermis (11) to be treated can be made of sapphire, quartz, industrial diamond or suchlike; the choice of the said latter materials being based on the degree of thermal conductivity required.

5. A device according to claim 1 **characterised by** the fact that the optical cooling unit (1) is kept watertight and has two orifices (5) and (6), for, respectively, the inlet and output of the cooling fluid contained within the cavity (2); the said orifices being connected to a circulation and cooling circuit for the said fluid.

6. A device according to claims 1 and 5, **characterised by** the fact that the temperature of the cooling fluid is maintained within a range from -10°C and +5°C.

7. A device according to claims 1, 5 and 6, **characterised by** the fact that the cooling fluid can be constituted solely of water, of a mixture of water and glycol or suchlike, or even of cryogenic gas.

8. A device according to claims 1, 5, 6 and 7, **characterised by** the fact that the flow of the cooling fluid inside the optical cooling unit (1) is continuous for the entire time the device (7) is in use.

9. A device according to claim 1, **characterised by** the fact that each of the additional optics (15) has its own distinctive focalising power.
